Europäisches Patentamt

European Patent Office

Office européen des brevets

(19)

(11) Numéro de publication : **0 457 685 A1**

(12) ## DEMANDE DE BREVET EUROPEEN

(21) Numéro de dépôt : 91401273.7

(22) Date de dépôt : 16.05.91

(51) Int. Cl.⁵ : **C12Q 1/04, C12Q 1/18, C12Q 1/68, C12N 1/20, A61K 39/02, C12P 21/08, C07H 21/00**

(30) Priorité : 18.05.90 FR 9006285

(43) Date de publication de la demande :
**21.11.91 Bulletin 91/47**

(84) Etats contractants désignés :
**AT BE CH DE DK ES GB GR IT LI LU NL SE**

(71) Demandeur : **INSTITUT PASTEUR**
**25-28, rue du Docteur Roux**
**F-75724 Paris Cédex 15 (FR)**

(72) Inventeur : **Montagnier, Luc**
**21 rue de Malabry**
**F-92350 Le Plessis Robinson (FR)**

Inventeur : **Blanchard, Alain**
**14, avenue de la Paix**
**F-92120 Montrouge (FR)**
Inventeur : **Di Rienzo, Anne Marie**
**61, rue Falguière**
**F-75015 Paris (FR)**
Inventeur : **Guetard, Denise**
**4B, rue Anselme Payen**
**F-75015 Paris (FR)**
Inventeur : **Rame, Véronique**
**66 rue St-Antoine**
**F-75004 Paris (FR)**

(74) Mandataire : **Desaix, Anne et al**
**Ernest Gutmann - Yves Plasseraud S.A., 67**
**bld.Haussmann**
**F-75008 Paris (FR)**

(54) **Nouveaux mycoplasmes - moyens pour détecter et caractériser des mycoplasmes in vitro.**

(57)    La présente invention concerne de nouveaux mycoplasmes mis en évidence chez des patients susceptibles d'être affectés par un SIDA.
    Elle vise aussi des moyens pour détecter in vitro la présence de mycoplasmes dans un fluide biologique, à l'aide d'un réactif spécifique du groupe des mycoplasmes, mais non spécifique d'espèces particulières au sein de ce groupe.
    L'invention concerne aussi des moyens pour tester la sensibilité de mycoplasmes à des antibiotiques.

EP 0 457 685 A1

L'invention concerne de nouveaux mycoplasmes, ainsi que des moyens pour les détecter et les caractériser.

Les mycoplasmes sont des agents infectieux pour l'homme souvent impliqués dans différentes pathologies. Il existe différents types de mycoplasmes différenciés selon les pathologies dans lesquelles ils sont présents.

On a déjà constaté dans le cas d'infection par un rétrovirus VIH (abréviation anglaise correspondante HIV : Human Immunodeficiency Virus) responsable du SIDA, que des mycoplasmes peuvent être mis en évidence dans certains tissus. Ainsi Lo et al ont détecté dans les tissus de certains patients atteints de SIDA, un mycoplasme désigné par M incognitus et qui présente une grande parenté avec M fermentans, un autre mycoplasme connu (Lo et al Am.J. trop. Med. Hyg 41 (5) 1989 pp 586 -600).

Pour détecter ce mycoplasme Lo et al ont eu recours d'une part à des anticorps et d'autre part à une sonde nucléotidide spécifique de M incognitus.

Contrairement à Lo et al qui ont émis l'hypothèse de la responsabilité d'un mycoplasme spécifique dans l'infection de patients ayant le SIDA et qui ont mis au point des moyens, notamment des sondes, spécifiques de ce mycoplasme pour détecter sa présence dans des tissus de malades, les inventeurs ont au contraire constaté que les mycoplasmes présents chez des patients, infectés par le VIH, peuvent appartenir à des types différents. Ils ont donc recherché la présence d'éventuels mycoplasmes dans le sang de patients infectés par un rétrovirus humain VIH ou atteints de SIDA avec des moyens non spécifiques d'un mycoplasme particulier.

Les inventeurs ont également identifié et caractérisé de nouveaux mycoplasmes, présents dans le sang de patients infectés par le VIH montrant ainsi que l'infection par VIH peut-être accompagnée par une infection par un ou plusieurs mycoplasmes différent(s) selon les patients.

Partant de ces constatations, les inventeurs ont élaboré des moyens destinés à détecter et isoler des mycoplasmes à partir d'échantillons de sang prélevés chez des patients infectés par VIH ou chez d'autres patients infectés par des mycoplasmes mais non contaminés par le VIH.

Afin de définir un traitement actif vis-à-vis de mycoplasmes déterminés, ils ont également mis au point des moyens pour tester leur sensibilité à des médicaments, en particulier à des antibiotiques. L'invention permet aussi de réaliser un suivi des malades infectés par des mycoplasmes et le cas échéant d'adapter le traitement médical à l'évolution de l'infection.

L'invention a donc pour objet de nouveaux mycoplasmes apparentés ou non à des catégories connues de mycoplasmes présents notamment chez des patients atteints par le VIH.

Un premier mycoplasme selon l'invention est le mycoplasme correspondant à la souche ber déposée à la Collection Nationale des Cultures de Microorganismes de Paris (CNCM) sous le numéro I-950 le 3 mai 1990 et à la NCIMB le 17 mars 1990 sous le numéro 402X3.

Un autre mycoplasme selon l'invention est M. fermantans N°8 déposé à la Collection Nationale des Cultures de Microorganismes de Paris (CNCM) sous le numéro I-949 le 3 mai 1990 et à la NCIMB le 17 mars 1990 sous le numéro 40284.

Les mycoplasmes selon l'invention sont encore caractérisés en ce que leur acide nucléique hybrident avec des séquences nucléotidiques des génomes de M. ber ou M. fermentans n° 8 dans les conditions exposées dans D. Amikam et al, Nucleic Acids Research (1982) vol. 10 n° 14, pp 4215-4522.

L'invention concerne aussi l'utilisation d'une sonde nucléotidique telle que décrite ci-dessus pour l'identification de mycoplasmes dont on souhaite identifier la nature dans un échantillon biologique d'un patient susceptible d'être affecté par un SIDA, notamment par mise en oeuvre des techniques de PCR, ou pour l'utilisation dans le suivi de traitement de ce patient infecté par les mycoplasmes.

L'invention vise aussi des sondes spécifiques du groupe des mycoplasmes mais non spécifiques d'espèces, pour la détection d'une infection par des mycoplasmes.

Les mycoplasmes précédemment décrits, isolés chez des patients atteints par le VIH, sont responsables de l'effet cytolytique induit par le VIH dans des lymphocytes ou dans des cellules de lignées lymphoides. Par conséquent, l'isolement et la caractérisation de mycoplasmes en particulier dans des échantillons sanguins de patients infectés par VIH, peuvent permettre d'agir sur la pathogénèse du SIDA.

La constatation par les inventeurs de la possibilité de détecter des mycoplasmes dans le sang avec des moyens non spécifiques d'espèces particulières dans le groupe des mycoplasmes leur a donné la possibilité de concevoir des moyens pour détecter, isoler et agir sur une infection par des mycoplasmes au niveau du sang. Les moyens ainsi élaborés par les inventeurs sont applicables non seulement aux cas de pathologie liée au virus VIH mais également à toute infection par des mycoplasmes, qui se traduit par leur présence dans le sang.

L'invention a donc pour objet un procédé de détection in vitro d'une infection par un VIH ou de son degré d'évolution chez un patient suspecté d'en être porteur caractérisé :

– par la mise en contact d'un réactif spécifique du groupe des mycoplasmes mais non spécifique d'espèces particulières au sein de ce groupe, avec une composition elle-même issue d'un fluide biologique prélevé chez ce patient, cette composition étant constituée par le fluide biologique lui-même ou résultant de l'isolement à partir de cet échantillon d'une fraction susceptible de contenir des mycoplasmes, ceux-ci ayant le cas échéant, fait l'objet d'une amplification préalable,

– par la détection à l'aide du susdit réactif de la présence de mycoplasmes au sein de la susdite composition.

Selon un mode de réalisation avantageux de l'invention, le fluide biologique à partir duquel est effectuée la détection, est un échantillon de sang et la composition suspectée de contenir les mycoplasmes comprend des éléments figurés du sang notamment les globules rouges ou les lymphocytes. Lorsque le fluide biologique est le sang, un procédé de détection particulier entrant dans la définition ci-dessus est caractérisé en ce que l'on réalise les étapes suivantes préalablement à la détection des mycoplasmes :

a) on traite l'échantillon de sang pour séparer les globules rouges qui constituent une fraction susceptible de contenir les mycoplasmes sur laquelle la détection est réalisée, des autres constituants du sang,

b) on sépare les globules rouges des mycoplasmes éventuels par sédimentation des globules rouges,

c) on réalise une ultracentrifugation du surnageant obtenu après sédimentation dans des conditions susceptibles de provoquer la sédimentation des composants qui, au sein du surnageant, sont porteurs des mycoplasmes et on récupère le culot d'ultracentrifugation, la susdite détection des mycoplasmes étant effectuée sur le culot.

Pour le traitement initial des globules rouges on peut procéder par centrifugation de l'échantillon de sang à 400 g pendant 30 minutes et lavage dans un milieu déterminé le cas échéant additionné d'antibiotiques inactifs sur les mycoplasmes, ce milieu de lavage étant par exemple un milieu RPMI 16-40 additionné de 10 % de sérum de veau foetal préalablement décomplémenté, par exemple par chauffage à 56°C pendant 30 mm, ce sérum ayant été testé pour l'absence de mycoplasmes.

Avantageusement la centrifugation des globules rouges est effectuée à 1000 g pendant 5 mn et l'ultracentrifugation du surnageant obtenu après centrifugation est effectuée à 245000 g pendant 60 mn.

Dans le cadre de la réalisation de la détection ci-dessus à partir des globules rouges la détection des mycoplasmes éventuellement présents comprend l'étape suivante :

– on inocule le culot d'ultracentrifugation obtenu selon ce qui précède, à un milieu de culture particulièrement adapté à la culture des mycoplasmes par exemple un milieu SP-4 enrichi selon ce qui est décrit par Tully et al (Science 1977 195, page 892-894 la détection des mycoplasmes étant alors effectuée dans ce milieu.

Dans ce cas la détection est réalisée directement après mise en contact de la composition suspectée de contenir les mycoplasmes, avec le milieu de culture spécifique des mycoplasmes, par exemple un milieu SP-4 enrichi comme ci-dessus.

Un autre mode de détection préféré à partir des globules rouges est caractérisé en ce que la détection de la présence de mycoplasmes comprend l'inoculation avec la susdite composition et en ce que l'on détecte le développement d'une infection des lymphocytes initialement sains par les mycoplasmes de la composition.

Dans ce cas les lymphocytes non contaminés inoculés par les mycoplasmes sont marqués par exemple avec de l'uracile radioactive, et le surnageant de culture est sédimenté à l'équilibre de densité dans un gradient de saccharose, avant de détecter l'incorporation de l'uracile dans les cultures, cette incorporation caractérisant la présence de mycoplasmes. D'une manière générale, la sédimentation est telle qu'elle permet de séparer les mycoplasmes des autres constituants.

Ce procédé peut présenter l'avantage par rapport à la croissance dans un milieu synthétique tel que le milieu SP-4 , d'être très rapide et ultra sensible. De plus cette technique de marquage à l'uracile présente une spécificité satisfaisante, dès lors que seuls les microorganismes peuvent incorporer l'uracile.

D'autres marqueurs peuvent remplacer l'uracile dès lors qu'ils possèdent la spécificité recherchée vis-à-vis des microorganismes.

Lorsque l'on recherche la présence de mycoplasmes à partir d'une culture de lymphocytes préalablement inoculés avec les mycoplasmes selon ce qui est décrit plus haut, on peut aussi détecter la présence de mycoplasmes par la mise en contact de la composition susceptible de contenir ces mycoplasmes, avec un réactif contenant au moins un antibiotique actif vis-à-vis des mycoplasmes notamment de la famille des quinolones, suivie de la détection d'une éventuelle réaction entre la composition ci-dessus et le ou les antibiotiques (s).

Outre les antibiotiques de la famille des quinolones on peut utiliser des macrolides ou des cyclines.

Selon un autre mode de réalisation de l'invention, la détection des mycoplasmes correspond à celle de leur acide nucléique. L'invention concerne à cet égard un procédé de détection in vitro, caractérisé en ce que la composition susceptible de contenir les mycoplasmes est traitée de façon à rendre l'ADN des mycoplasmes accessible au réactif comprenant une sonde contenant une séquence d'ADN elle-même dérivée d'une séquence hautement conservée dans les ARN ribosomiques caractéristiques de mycopolasmes.

3

Dans le cas de la recherche de l'ADN on peut opérer à partir du plasma ou de lymphocytes infectés tels qu'obtenus à partir d'un fluide biologique prélevé chez un patient.

Préalablement à la séparation de l'ADN, une étape d'amplification peut être souhaitable, par exemple dans un milieu SP-4, jusqu'à obtention d'une concentration en mycoplasmes de $10^8$/ml.

Pour rendre l'ADN accessible, on traite avantageusement la culture de mycoplasmes par centrifugation et le culot de centrifugation récupéré est digéré avec un mélange de protéinase K et de SDS. Ainsi pour détecter l'ADN de mycoplasmes, un procédé avantageux selon l'invention est réalisé comme suit :

– on centrifuge la culture contenant les mycoplasmes, dans des conditions permettant la sédimentation des mycoplasmes et le cas échéant après une étape d'amplification sur un milieu de culture spécifique des mycoplasmes,

– on traite le culot de centrifugation afin de recueillir l'ADN, par exemple avec un mélange de protéinase K et de SDS,

– on met en contact l'ADN ainsi obtenu avec le réactif comprenant au moins une sonde contenant une séquence d'ADN elle-même dérivée d'une séquence hautement conservée dans les ARN ribosomiques caractéristiques des mycoplasmes.

Pour mettre en oeuvre le procédé de détection de l'ADN des mycoplasmes, différentes sondes peuvent être utilisées. A titre d'exemple on cite la sonde pMC5 correspondant à l'opéron de l'ARN ribosomique de M. Capricolum (D. Amikam et al, Ribosomal RNA genes in Mycoplasma, Nucleic Acids Research -1982- vol 10 n° 14 pp 4215-4222). La réaction d'hybridation a lieu après digestion par les enzymes Eco RI et HindIII de l'ADN à détecter.

Après avoir mis en évidence la présence de mycoplasmes dans la composition testée, on peut également choisir d'utiliser des sondes présentant une spécificité élevée vis-à-vis d'une espèce de mycoplasmes soupçonnés d'être responsables de l'infection, pour identifier plus précisément la nature du mycoplasme et prévoir ainsi un traitement plus spécifique. Par exemple dans le cas d'une infection à mycoplasmes liée à "une infection par le virus VIH, on pourra avantageusement mettre en oeuvre des sondes telles qu'obtenues à partir des souches de mycoplasmes M fermantans N° 8 et de la souche ber.

L'invention a également pour objet l'application de ces procédés pour la détection de toute infection à mycoplasmes, notamment à partir du sang d'un patient. Entre également dans le cadre de l'invention un test pour rechercher in vitro la sensibilité à un ou plusieurs antibiotiques donnés, de mycoplasmes préalablement isolés à partir d'un échantillon biologique d'un patient susceptible d'être affecté par un SIDA, caractérisé en ce que,

– la culture cellulaire contaminée avec le ou les mycoplasme (s) isolé (s) est incubée avec des doses croissantes de différents antibiotiques,

– cette culture est marquée par exemple avec de l'uracile radioactive,

– la radioactivité de chaque fraction est déterminée après sédimentation du surnageant dans des conditions permettant de séparer les mycoplasmes des autres constituants.

Avantageusement,le surnageant est sédimenté à l'équilibre de densité dans un gradient de saccharose, et la radioactivité de chaque fraction après précipitation, par exemple avec 5 % de TCA, est déterminée.

Le cas échéant la surface des pics correspondant à la densité de mycoplasmes par rapport à un témoin non traité par les antibiotiques est calculée, afin de déterminer le pourcentage d'inhibition de la croissance de la culture.

La mise en oeuvre de ce test permet de rechercher parmi des antibiotiques connus si leur activité vis-à-vis des mycoplasmes présente un intérêt dans le traitement de malades atteints par ces mycoplasmes. Des antibiotiques particulièrement utiles pour le traitement des infections à mycoplasmes sont par exemple des antibiotiques de la famille des quinolones, des cyclines ou des macrolides.

Le test de sensibilité ci-dessus peut être conduit également pour déterminer quelles sont les doses appropriées devant être administrées pour la réalisation du traitement. Ce test peut également avoir un intérêt dans le suivi de l'efficacité d'un traitement donné avec un antibiotique choisi.

Le test de sensibilité précédemment décrit peut encore être utilisé pour rechercher l'efficacité éventuelle de nouveaux antibiotiques vis-à-vis des mycoplasmes détectés, responsables d'une pathologie particulière.

D'autres caractéristiques et avantages de l'invention apparaissent dans les figures et les exemples qui suivent.

L'invention vise aussi une composition immunogène caractérisée en ce qu'elle contient au moins un mycoplasme selon l'invention. Elle concerne aussi des anticorps en particulier des anticorps monoclonaux caractérisés en ce qu'ils reconnaissent les mycoplasmes de l'invention. Ces anticorps sont préparés par les techniques conventionnelles par exemple par fusion de cellules spléniques d'un animal préalablement immmunisé avec un mycoplasme donné, avec un myclome déterminé, pour former un hybridome, culture de l'hybridome et récupération des anticorps formée.

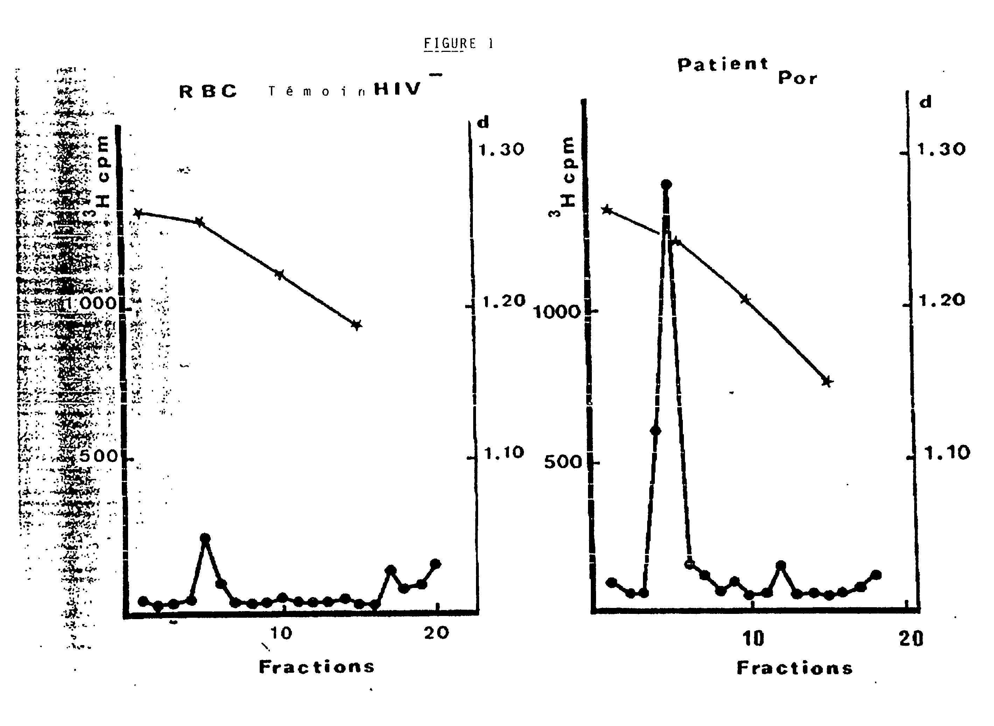

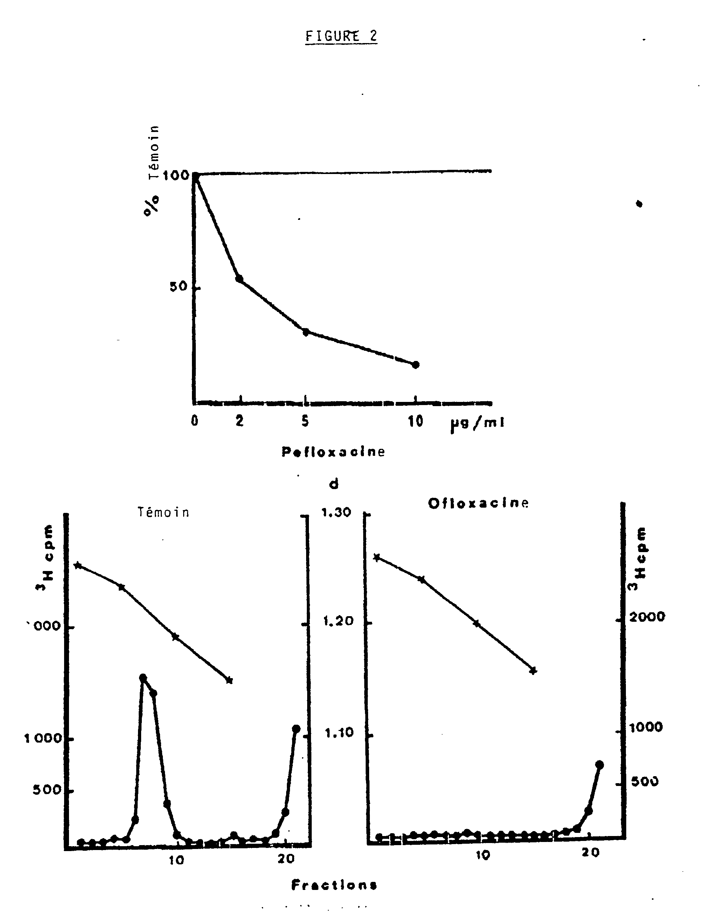

Des techniques sont encore décrites ci-après en rapport avec les légendes des figures 1 et 2.

## 1/ Procédés d'isolement

10 ml de sang du patient sont prélevés sous héparine et centrifugés sur une couche de Percoll (Seromed) à 400 g pendant 30 minutes.

a) Le culot de globules rouges est lavé en milieu RPMI 16-40 additionné de 10% de sérum de veau (préalablement décomplémenté par chauffage à 56° pendant 30 minutes et préalablement testé pour l'absence de mycoplasmes) et additionné d'antibiotiques non actifs sur les mycoplasmes (pénicilline 100 U/ml, streptomycine 100 Ug/ml). 50 ml de globules rouges respendus dans le milieu RPMI 16-40 sont incubés dans le même milieu 18 heures à 37°C à raison de $10^8$ globules rouges/ml. Les globules rouges sont ensuite sédimentés par centrifugation à 1000 g pendant 5 minutes, et le surnageant est ultracentrifugé à 245.000 g pendant 60 minutes.

Le culot pouvant éventuellement contenir les mycoplasmes est resuspendu dans 1 ml du milieu de culture et inoculé d'une part à des tubes de milieu SP-4 (Science 1977, 195, 892-894 Tully et al), milieu synthétique enrichi pour la culture des mycoplasmes, (), et d'autre part à des cultures de lymphocytes de donneurs normaux séronégatifs pour VIH et non contaminés par des mycoplasmes, stimulés par la PHA et cultivés en milieu RPMI 16-40 complet additionné de 20 U/ml d'interleukine 2.

Une partie des globules rouges est également gardée pour une mise en évidence directe des mycoplasmes selon les techniques décrites plus loin.

b) La bande de cellules blanches mononucléées du gradient Percoll est également lavée par centrifugation et une partie est mise en culture en milieu RPMI 16-40 complet + Il2 après trois jours d'activation à la PHA comme décrit plus haut. Une autre partie est directement inoculée dans des tubes de culture SP-4.

c) Enfin, 3 ml de plasma sont ultracentrifugés à 245.000 g pendant 60 minutes et le culot conservé pour l'extraction d'ADN.

## 2/ Détection des mycoplasmes et tests de sensibilité aux antibiotiques

Outre les techniques conventionnelles (croissance en milieux synthétiques, tel le milieu SP-4) , on utilise la technique suivante, rapide et ultrasensible :

Les lymphocytes activés mis en culture (voir plus haut) sont marqués 3 heures avec de l'uracile tritiée (20 µCi/ml, Amersham). Le surnageant est sédimenté à l'équilibre de densité dans un gradient de saccharose 70-20%, 1 hr à 45.000 tours/mn dans un rotor SW56 (tampon PBS + 2% sérum de veau foetal). Seuls des microorganismes peuvent incorporer l'uracile. Les mycoplasmes forment une bande de densité variant de 1,18 à 1,24 (Fig.1). Le marquage est inhibé par des antibiotiques actifs sur les mycoplasmes, tels les quinolones ou les tétracyclines.

Les mycoplasmes peuvent être passés en série sur d'autres lymphocytes stimulés ou sur des lignées lymphoides, telles la lignée CEM, (Clone 13) celle-ci ayant été débarassée de mycoplasmes contaminants éventuels par un traitement prolongé sous antibiotiques.

Pour déterminer rapidement la sensibilité aux antibiotiques d'un mycoplasme ainsi isolé, on procède de la manière suivante :

Des cultures cellulaires contaminées avec le mycoplasme sont incubées pendant 48 heures avec des doses croissantes de différents antibiotiques (cyclines, quinolones, macrolides). Puis on fait comme ci-dessus un marquage de 3 heures avec de l'uracile tritiée et une sédimentation à l'équilibre en gradient de saccharose du surnageant. La radioactivité de chaque fraction est déterminée après précipitation par 5 % de TCA. On calcule la surface des pics correspondant à la densité des mycoplasmes ( > 1,18) par rapport au témoin non traité par des antibiotiques, et on peut ainsi déterminer des pourcentages d'inhibition. La figure 2 est représentative d'une telle détermination effectuée à partir d'un mycoplasme isolé à partir d'un malade.

## 3/ Clonage moléculaire de l'ADN de mycoplasmes et utilisation des sondes pour le diagnostic

Plusieurs types de mycoplasmes isolés à partir de malades atteints de SIDA ou de porteurs asymptomatiques se sont révélés être différents par leurs propriétés moléculaires et biologiques des mycoplasmes décrits jusqu'à présent chez l'homme.

On a pu en particulier différencier une nouvelle espèce de mycoplasmes, isolée notamment de deux malades n'ayant pas eu de relations entre eux, par l'analyse des fragments de restriction des gènes ribosomiques.

Le mycoplasme a été isolé à partir des cultures de lymphocytes d'un malade atteint de SIDA, ber. La culture de mycoplasmes a été amplifiée en milieu synthétique SP-4, jusqu'à une concentration de $10^8$/ml. 200 ml sont centrifugés et conservés à - 80°C après un lavage en tampon PBS. Après décongélation rapide, le culot est immédiatement digéré par un mélange de protéinase K et SDS (150 mM NaCl, 10 mM Tris/HCl pH 8,0, 10 mM EDTA, 0.5% mg/ml Protéinase K), à raison de 0,1 ml de mélange par $10^8$ cellules pour une durée de 1 heure à 55°C suivie de 6 heures à 37°C. Environ 300 µg d'ADN sont récupérés habituellement à partir de $10^{10}$ cellules. Cet ADN est analysé par les techniques de "Dot-blot", analyse de restriction, Southern transfert et hybridation, dont on trouvera une description détaillée dans la référence Amikam et al (J of Bact. 1984 vol 158 p 376-378).

La sonde utilisée, pMC5, correspondant à l'opéron de RNA ribosomique de M. capricolum (Amikam et al Nucl. Ac. Res. 1982, vol. 10, N° 14 pp 4215-4222) est capable de reconnaitre d'une façon très efficace les séquences ribosomiques correspondantes des mycoplasmes, après digestion par les enzymes Eco RI et Hind III.

Les résultats d'analyse en gel d'agarose par Southern après digestion par ces deux enzymes, des opérons ribosomiques de différents mycoplasmes humains isolés indiquent ce qui suit : le mycoplasme N° 8 montre un profil identique à celui des deux souches de M. fermentans et de M. incognitus (ce dernier n'étant en fait qu'une souche de M. fermantans) et est par conséquent appelé M. fermentans N° 8. Par contre, les ADN des mycoplasmes ber et vuc montrent un profil identique entre eux et à celui donné par l'espèce M. pirum, indiquant que les mycoplasmes ber et vuc sont des souches de l'espèce M. spirum. Les profils des mycoplasmes ber et vuc sont différents de ceux donnés par les mycoplasmes suivants : M. orale, M. capricolum, M. pneumoniae, M. arginini, M. gallisepticum, A. laidlawii, A. axanthum, A. granularum, Ureaplasma urealyticum, Spiroplasma citri, M. genitalium, M. hominis, M. fermentans (Tableau N°1).

L'ADN de Mycoplasma fermentans N° 8 et celui de M. ber ont été clonés dans un plasmide Blue script II (Stratagène), après digestion par Eco RI. 150 clones de 1000 à 2000 bp ont été obtenus et ceux montrant une absence d'hybridation avec les régions les plus conservées des mycoplasmes ont été retenus. Des clones moléculaires spécifiques de M. ber et M. fermentans ont ainsi été obtenus et séquencés. Ces séquences permettent de déduire des oligonucléotides amorces spécifiques pour la PCR (réaction d'amplification génique ou polymerase chain reaction). On peut ainsi appliquer la PCR et les Dot blots (empreintes) au suivi thérapeutique de malades infectés par ce type de mycoplasmes et recevant un traitement antibiotique destiné à les guérir de cette infection. Les propriétés biologiques de M ber et M. fermentans N° 8 sont résumées dans le tableau 2 suivant.

Tableau 2

| Organisme | ECORI | | HinOII | |
|---|---|---|---|---|
| | N° des bandes d'hybrida-tion | Taille du segment chromosomal hybridé (kb) | N° des bandes d'hybrida-tion | Taille du segment chromosomal hybridé (kb) |
| *Mycoplasma orale (ATCC 23714) | 1 | 5 | NA | NA |
| *Mycoplasma capricolum (ATCC 27343) | 2 | 4.8 ; 2 | NA | NA |
| **Mycoplasma pneumoniae (strain FH and M129-B16) | 1 | 9 | 3 | 85 ;1.35 ;0.75 |
| *Mycoplasma arginini (G-230) | 2 | 4 ; 7 | NA | NA |
| *Mycoplasma gallisepticum (strain S6) | 1 | 8 | NA | NA |
| *Mycoplasma gallisepticum (strain A5969) | 3 | 8 : 6.2; 9 | NA | NA |
| *Acholeplasma laidlawii (oral strain) | 2 | 5.2 ; 8.8 | NA | NA |
| *Acholeplasma axanthum | 2 | 6.4 ; 7 | NA | NA |
| *Acholeplasma granularum | 2 | 8.9 ; 9.6 | NA | NA |
| *Ureaplasma urealyticum | 4 | 3.5,6.2;8.2;12 | NA | NA |
| *Spiroplasma citri (Maroc R8A2) | 1 | 13 | NA | NA |
| *Mycoplasma genitalium | 2 | ca.2.4;ca.4.8 | NA | NA |
| *Mycoplasma hominis | 3 | 1.6;5.38;13 | 2 | 3.5 ;10.4 |
| *Mycoplasma fermentans (PG-18) | 3 | 1.55;4.4;6.35 | 3 | 1.46;2.63;3 |
| *Mycoplasma fermentans (K7) | 3 | 1.52;4.4;6.2 | 3 | 1.43;2.6;3 |
| *Mycoplasma incognitus | 3 | 1.47;3.67;5.8 | 3 | 1.43;2.42;2.8 |
| *Mycoplasma pirum | 3 | 2.3;3.65;5.45 | 3 | 1.25;2.42;1.8 |
| *Ber | 3 | 2.3;3.65;5.45 | 3 | 1.25;1.35;1.8 |
| *Vuc | 3 | 2.3;3.65;5.45 | 3 | 1.25;1.35;1.8 |
| *No 8 | 3 | 1.49;4.16;4.46 | 3 | 1.44;2.55;3 |

Tableau 1 : Schéma d'hybridation après digestion avec HindIII et/ou EcoRI de l'ADN de différents génomes de mycoplasmes et de mycoplasmes isolés à partir de patients ayant le SIDA en utilisant pMC5 contenant de l'ARNr de M. capricolum comme sonde. NA : non disponible

EP 0 457 685 A1

Tableau 2

### PROPRIETES BIOLOGIQUES DES MYCOPLASMES n°8 et b6r:

| Croissance sur SP-4 | Durée de la culture* | Fermentation du glucose | Hydrolyse de l'arginine | Sensible à la la digitonine | Colonies en "oeuf sur le plat" |
|---|---|---|---|---|---|
| oui | 7 jours | oui | oui | oui | non |
| oui | 5 jours | oui | oui | oui | non |

* Temps mis par la culture pour faire virer le rouge de phénol au jaune avec un ensemencement au 1/10 du volume total.

Légende de la figure 1 :

Détection de mycoplasmes dont l'existence est présumée par la technique du gradient de densité à l'uracyle.

10-15 ml de sang héparinisé sont centrifugés à 400 g dans un gradient de Percoll pendant 30 minutes. Le culot de globules rouges est lavé dans un milieu de culture (RPMI 16-40 + 10% de sérum de veau foetal décomplémenté + pénicilline 100 U/ml, Streptomycine 50 μg/ml).

Les érythrocytes lavés sont incubés pendant 18 heures à 37°C dans le même milieu (auquel avait été ou est ajouté 20 U de II 2) à raison de $10^8$ T lymphocytes d'un donneur sain par ml, et stimulés par la de 5-$10^5$/ml de PHA.

La coculture est ensuite incubée pendant 3 heures en présence de 20 Ci/ml de $^3$H uracile (AMERSHAM 44 Ci/ml).

Après addition de 3mM d'azide de sodium, les cellules sont sédimentées à 1000 g pendant 5'. Une partie du surnageant clarifié (1 ml) est sédimentée dans un gradient de sucrose 20-70% dans un tampon PBS complété avec du sérum de veau foetal à 2% à 245000g pendant 60 minutes dans un rotor BECKMAN SW56.

Des fractions aliquotes des fractions collectées ont été prélevées pour des mesures de densité avec un réfractomètre. Le restant est précipité avec du TCA à 5% et soumis à comptage dans un spectromètre à scintillation (LKB).

Toutes les centrifugations sont effectuées, de préférence, à 40°C, dans des conditions stériles. Les solutions de sucrose sont autoclavées.

Des témoins sont effectués avec seulement le gradient de sucrose, et seulement avec le surnageant des T lymphocytes. Aucun pic n'a été observé.

Légende de la figure 2 :

Sensibilité aux antibiotiques de M. pirum isolé à partir d'un sérum de patient (BER).

Des cellules CEM/13, préalablement cultivées pendant trois semaines avec 0,5 μg/ml de MRA (abréviation de l'expression anglaise "Mycoplasma Removal Agent" = Agent d'élimination de mycoplasmes) sont testées pour leur absence de contamination par des mycoplasmes. Elles sont utilisées pour faire des cultures de M.pirum.

Après une semaine de cultures, les cellules sont incubées en présence d'antibiotique pendant 48 heures, puis marquées pendant 3 heures avec du $^3$H uracyle comme décrit dans la figure 1.

Le surnageant des cellules est analysé dans un gradient de sucrose, comme également indiqué à la figure 1.

La surface du pic de mycoplasmes (fractions à des densités contenues dans la gamme 1,18 à 1,24) est mesurée et exprimée en % vis-à-vis des témoins non traités.

En haut : courbe dose réponse de la pefloxacine.

En bas : deux exemples d'analyse dans un gradient de sucrose : témoins non traités (à gauche) et cellules traitées avec 5 μg/ml d'ofloxacine.

La courbe de la partie gauche correspond à un donneur sain : voir petit pic à p = 1,24.

La courbe à droite correspondant à des cellules de sérum d'un malade du SIDA, infecté par le virus à l'occasion d'un contact sexuel.

Ref. pour pMC5 :

D. Amikam, S. Razin and G. Glaser. Ribosomal RNA genes in Mycoplasma - Nucleic Acids Research (1982) vol. 10 n° 14 pp4215-4222.

Antibiogramme/mycoplasmes :

In : Methods in Mycoplasmology, vol. II. "Antibiotic sensitivity testing of mycoplasmas" by L.B. Senterfit. J.G. Tully, S. Razin eds. Academic Press, Inc. New York.

## Revendications

1/ Procéde de détection in vitro d'une infection par un VIH ou de non degré d'évolution, chez un patient suspecté d'en être porteur en particulier caractérisé :
- par la mise en contact d'un réactif spécifique du groupe des mycoplasmes mais non spécifique d'espèces particulières au sein de ce groupe, avec une composition elle-même issue d'un fluide biologique prélevé chez ce patient, cette composition étant constituée par le fluide biologique lui-même ou résultant de l'isolement à partir de cet échantillon d'une fraction susceptible de contenir des mycoplasmes, ceux-ci ayant le cas échéant, fait l'objet d'une amplification préalable,

– par la détection à l'aide du susdit réactif de la présence de mycoplasmes au sein de la susdite composition.

2/ procédé de détection in vitro selon la revendication 1, caractérisé en ce que le fluide biologique est le sang et en ce que la composition comprend des éléments figurés du sang..

3/ procédé de détection in vitro selon la revendication 2 caractérisé en ce que l'on réalise les étapes suivantes préalablement à la détection des mycoplasmes :

a) on traite l'échantillon de sang pour séparer les globules rouges qui constituent une fraction susceptible de contenir les mycoplasmes sur laquelle la détection est réalisée, des autres constituants du sang,

b) on sépare les globules rouges des mycoplasmes éventuels par sédimentation des globules rouges,

c) on réalise une ultracentrifugation du surnageant obtenu après sédimentation dans des conditions susceptibles de provoquer la sédimentation des composants qui, au sein du surnageant, sont porteurs des mycoplasmes et on récupère le culot d'ultracentrifugation, la susdite détection du mycoplasme étant effectuée sur le culot.

4/ procédé de détection in vitro selon la revendication 3, caractérisé en ce que l'étape de la détection des mycoplasmes éventuellement présents comprend l'étape suivante :

– on inocule le culot d'ultracentrifugation, à un milieu de culture particulièrement adapté à la culture des mycoplasmes par exemple un milieu SP-4 enrichi pour la culture des mycoplasmes, la détection du mycoplasme étant alors effectuée dans ce milieu.

5/ Procédé de détection in vitro selon l'une quelconque des revendications 1 à 4 caractérisé en ce que la détection est réalisée directement après mise en contact de la composition suspectée de contenir les mycoplasmes, avec un milieu de culture spécifique des mycoplasmes, par exemple un milieu SP-4 enrichi , la détection des mycoplasmes étant alors effectuée dans ce milieu.

6/ Procédé de détection in vitro selon l'une quelconque des revendications 1 à 4, caractérisé en ce que la détection de la présence de mycoplasmes avec la susdite composition comprend l'inoculation avec la susdite composition et en ce que l'on détecte le développement d'une infection des lymphocytes initialement sains par les mycoplasmes de la composition.

– on inocule le culot d'ultracentrifugation à des lymphocytes sains, non contaminés par des mycoplasmes,

7/ Procédé de détection in vitro selon l'une quelconque des revendications 1 à 6 caractérisé en ce que la détection de la présence de mycoplasmes est réalisée par marquage avec de l'uracile radioactive, suivi d'une observation de l'incorporation de l'uracile par les mycoplasmes.

8/ Procédé de détection in vitro selon l'une quelconque des revendications 1 à 9 caractérisé en ce que la détection de la présence de mycoplasmes est réalisée par la mise en contact de la composition susceptible de contenir ces mycoplasmes, avec un réactif contenant au moins un antibiotique actif vis-à-vis des mycoplasmes notamment de la famille des quinolones, suivie de la détection d'une éventuelle réaction entre la composition ci-dessus et le ou les antibiotiques (s).

9/ Procédé de détection in vitro selon l'une quelconque des revendications 1 à 9, caractérisé en ce que la composition susceptible de contenir les mycoplasmes est traitée de façon à rendre l'ADN des mycoplasmes accessible au réactif comprenant une sonde contenant une séquence d'ADN elle-même dérivée d'une séquence hautement conservée dans les ARN ribosomiques caractéristiques de mycopolasmes.

10/ Procédé de détection in vitro selon la revendication 9 caractérisé en ce que la composition du fluide biologique susceptible de contenir les mycoplasmes est le plasma et en ce que la détection de l'ADN des mycoplasmes est réalisée à partir du culot obtenu après ultracentrifugation du plasma dans des conditions susceptibles de provoquer la sédimentation des mycoplasmes.

11/ Procédé de détection in vitro selon la revendication 9 caractérisé en ce que la composition du fluide biologique susceptible de contenir les mycoplasmes est une culture de lymphocytes infectés par le virus VIH.

12/ Procédé de détection in vitro selon la revendication 11 caractérisé en ce que la détection est réalisée comme suit :

– on centrifuge la culture contenant les mycoplasmes, dans des conditions permettant la sédimentation des mycoplasmes et le cas échéant après une étape d'amplification sur un milieu de culture spécifique des mycoplasmes,

– on traite le culot de centrifugation afin de recueillir l'ADN, par exemple avec un mélange de protéinase K et de SDS,

– on met en contact l'ADN ainsi obtenu avec le réactif contenant une sonde contenant une séquence d'ADN elle-même dérivée d'une séquence hautement conservée dans les ADN ribosomiques caractéristiques des mycoplasmes.

**13/** Procédé de détection selon l'une quelconque des revendications 9 à 12, caractérisé en ce que la sonde d'hybridation mise en oeuvre est la sonde pMC5 correspondant à l'opéron de l'ARN ribosomique de <u>M. Capricolum</u> et en ce que la réaction d'hybridation a lieu après digestion par les enzymes <u>Eco</u> RI et <u>Hind</u>III de l'ADN à détecter.

**14/** Test pour rechercher <u>in vitro</u> la sensibilité à un ou plusieurs antibiotiques donnés, de mycoplasmes préalablement isolés à partir d'un échantillon biologique d'un patient susceptible d'être affecté par un SIDA, caractérisé en ce que ,

– des cultures cellulaires contaminées avec le ou les mycoplasme (s) isolé (s) sont incubées avec des doses croissantes de différents antibiotiques,

– cette culture est marquée par exemple avec de l'uracile radioactive,

– la radioactivité de chaque fraction est déterminée après sédimentation du surnageant dans des conditions permettant de séparer les mycoplasmes des autres constituants.

– le cas échéant la surface des pics correspondant à la densité de mycoplasmes par rapport à un témoin non traité par les antibiotiques est calculée, afin de déterminer le pourcentage d'inhibition.

**15/** Souche de mycoplasme caractérisée en ce qu'il s'agit de la souche ber déposée à la CMCM sous le n° I-950 le 3 mai 1990 et à la NCIMB sous le n° 40283 le 17 Mars 1990.

**16/** Souche de mycoplasme caractérisée en ce qu'il s'agit de la souche M. fermantens N°8 déposée à la CNCM sous le n° I-949 le 3 mai 1990 et à la NCIMB sous le n° 40284 le 17 Mars 1990.

**17/** Acide nucléique de mycoplasme, caractérisé en ce qu'il répond à la séquence nucléotidique de M fermantans n° 8 déposée à la CNCM sous le n° I-949 ou à la séquence nucléotidique de M ber déposée à la CNCM sous le n° I-950, ou en ce qu'il hybride avec cette séquence dans les conditions stringentes avec le génome de M.ber ou M.fermantans.

**18/** Utilisation d'une sonde nucléotidique dérivée d'un génome de mycoplasme, pour la détection de mycoplasmes dans un échantillon biologique d'un patient susceptible d'être affecté par un SIDA, notamment par les techniques de PCR, ou pour l'utilisation dans le suivi du traitement de ce patient infecté par des mycoplasmes.

**19/** Composition immunogène caractérisée en ce qu'elle comprend au moins un mycoplasme selon l'une quelconque des revendications 15 ou 16.

**20/** Anticorps monoclonaux caractérisés en ce qu'ils sont dirigés contre les mycoplasmes selon l'une quelconque des revendications 15 ou 16.

FIGURE 1

RBC Témoin HIV⁻     Patient Por

## FIGURE 2

Office européen
des brevets

## RAPPORT DE RECHERCHE EUROPEENNE

Numero de la demande

EP 91 40 1273

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | EP-A-0 250 662 (THE REGENTS OF THE UNIVERSITY OF CALIFORNIA) * Revendications 1,3; colonne 5, lignes 7-43 * --- | 1,4,5,9 ,11,13, 14,18 | C 12 Q 1/04 C 12 Q 1/18 C 12 Q 1/68 C 12 N 1/20 A 61 K 39/02 C 12 P 21/08 C 07 H 21/00 |
| D,Y | AMERICAN JOURNAL OF TROPICAL MEDICINE AND HYGIENE, vol. 41, no. 5, novembre 1989, pages 586-600, US; S.-C. LO et al.: "Virus-like infections agent (VLIA) is a novel pathogenic mycoplasma: Mycoplasma incognitus" * Document entier * --- | 1,4,5,9 ,11,13, 14,18 | |
| A | IDEM --- | 16,17, 19,20 | |
| D,Y | NUCLEIC ACIDS RESEARCH, vol. 10, no. 14, 1982, pages 4215-4222, Oxford, GB; D. AMIKAM et al.: "Ribosomal RNA genes in mycoplasma" * Document entier * --- | 1,4,5,9 ,11,13, 14,18 | |
| A | US-A-4 387 161 (G.J. McGARRITY) * Revendication 1 * --- | 5,6 | |
| A | PATHOLOGIE - BIOLOGIE, vol. 25, no. 8, octobre 1977, pages 541-546; M. LABORDE: "Etude de la sensibilité aux antibiotiques de 34 souches de mycoplasmes a grande colonies" * Document en entier * --- -/- | 14 | |

DOMAINES TECHNIQUES RECHERCHES (Int. Cl.5)

C 12 Q
G 01 N
C 12 P
C 12 R

Le présent rapport a été établi pour toutes les revendications

| Lieu de la recherche | Date d'achèvement de la recherche | Examinateur |
|---|---|---|
| LA HAYE | 01-07-1991 | GRIFFITH G. |

Office européen
des brevets

**RAPPORT DE RECHERCHE EUROPEENNE**

Numero de la demande

EP   91 40 1273

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | CHEMICAL ABSTRACTS, vol. 80, no. 21, 1974, page 192, abrégé no. 117850v, Columbus, Ohio, US; E.L. SCHNEIDER et al.: "Incorporation of tritium-labeled uridine and tritium-labeled uracil into RNA. Simple technique for the detection of mycoplasma contamination of cultured cells", & EXP. CELL. RES. 1974, 84(1-2), 311-18 * Abrégé * | 1,4,5,9 ,11,13, 14,18 | |
| | | | **DOMAINES TECHNIQUES RECHERCHES (Int. Cl.5)** |

Le présent rapport a été établi pour toutes les revendications

| Lieu de la recherche | Date d'achèvement de la recherche | Examinateur |
|---|---|---|
| LA HAYE | 01-07-1991 | GRIFFITH G. |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
............................................................
& : membre de la même famille, document correspondant

EPO FORM 1503 03.82 (P0402)